(19) Europäisches Patentamt
European Patent Office
Office européen des brevets

(11) EP 2 077 106 A1

# (12) EUROPEAN PATENT APPLICATION

published in accordance with Art. 153(4) EPC

(43) Date of publication:
**08.07.2009 Bulletin 2009/28**

(21) Application number: **07830630.5**

(22) Date of filing: **26.10.2007**

(51) Int Cl.:
***A61K 9/113*** (2006.01) ***A61K 8/04*** (2006.01)
***A61K 8/30*** (2006.01) ***A61K 31/135*** (2006.01)
***A61K 31/138*** (2006.01) ***A61K 31/196*** (2006.01)
***A61K 31/352*** (2006.01) ***A61K 31/375*** (2006.01)
***A61K 31/401*** (2006.01) ***A61K 31/4365*** (2006.01)
***A61K 31/445*** (2006.01) ***A61K 31/502*** (2006.01)
***A61K 31/55*** (2006.01) ***A61K 31/554*** (2006.01)
***A61K 31/704*** (2006.01) ***A61K 47/02*** (2006.01)
***A61K 47/12*** (2006.01) ***A61K 47/18*** (2006.01)
***A61K 47/26*** (2006.01)

(86) International application number:
**PCT/JP2007/070896**

(87) International publication number:
**WO 2008/053799 (08.05.2008 Gazette 2008/19)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.10.2006 JP 2006292426**

(71) Applicant: **Controlled Lipo Techs, Inc.
Miyazaki-shi, Miyazaki 880-0122 (JP)**

(72) Inventors:
- **TERAO, Toshimitsu
Naruto-shi
Tokushima 772-0015 (JP)**
- **IMAGAWA, Takashi
Kobe-shi
Hyogo 651-1123 (JP)**
- **YANAGIE, Hironobu
Tokyo 106-0032 (JP)**
- **ERIGUCHI, Masazumi
Tokyo 141-0021 (JP)**

(74) Representative: **HOFFMANN EITLE
Patent- und Rechtsanwälte
Arabellastraße 4
81925 München (DE)**

## (54) W/O/W EMULSION COMPOSITION

(57) The present invention provides a W/O/W multiple emulsion composition composed of an internal aqueous phase, an oil phase, and an external aqueous phase, the internal aqueous phase containing an ionic physiologically active substance and a physiologically acceptable compound having a molecular weight of 1,000 or less and generating a polyvalent counterion with two or more valencies for the ionic physiologically active substance. The W/O/W emulsion composition of the present invention not only can stably encapsulate a useful substance in its internal aqueous phase at a high encapsulation ratio, but also has high safety.

EP 2 077 106 A1

## Description

TECHNICAL FIELD

The present invention relates to a W/O/W emulsion composition.

BACKGROUND ART

W/O/W emulsions that are liquid microcapsules have an aqueous phase as an external phase in which water-in-oil (W/O) droplets are dispersed.

W/O/W emulsions can have a various applications in food, drugs, cosmetics, etc., by enclosing useful substances inside the W/O droplets. W/O/W emulsions are also usable as intermediates in production processes. In order to take adequate advantage of W/O/W emulsions in these applications, it is important to be able to effectively suppress the leakage of a useful substance from the W/O droplets, and to control the release rate of the useful substance.

The encapsulation ratio of a useful substance in a W/O/W emulsion greatly depends on the concentration balance of the dissolved useful substance between the internal aqueous phase and the external aqueous phase. Generally, as the concentration of the useful substance in the internal aqueous phase increases, the concentration of the useful substance in the external aqueous phase also increases. It is thus difficult to maintain a high encapsulation ratio in the internal aqueous phase, causing a large amount of leakage of the useful substance from the W/O droplets to the external aqueous phase during the production or storage of the W/O/W emulsion.

In order to solve this problem, Patent Document 1, described below, suggests a method of producing an emulsion by adding an ionic drug and a water-soluble polymer capable of forming a complex with the foregoing drug, to the internal aqueous phase.

In this method, a water-soluble polymer is added to the internal aqueous phase so as to form a complex with an ionic drug; however, W/O/W emulsions containing a water-soluble polymer are difficult to use as injectable solutions. This is because water-soluble polymers cause an increase in the viscosity of an injectable solution, making the emulsification of W/O droplets difficult; additionally, due to the nature of polymer compounds, water-soluble polymers remain in the vehicle without leaking, and possibly remain in the body for a long period of time. Accordingly, the W/O/W emulsion of Patent Document 1 is presumably designed for use as a drug for external use.

For these reasons, further improvements are required to enable the effective use of W/O/W emulsions as injectable solutions, as well as suppressing of the leakage of useful substances from W/O droplets to the external aqueous phase effectively and more safely.

Patent Document 1: Japanese Unexamined Patent Publication No. 2005-97292

DISCLOSURE OF THE INVENTION

Problems to be Solved by the Invention

The present invention has been accomplished in view of the current state of the above-mentioned prior art. The primary object of the present invention is to provide a W/O/W emulsion composition that can stably contain a useful substance in the internal aqueous phase at a high encapsulation ratio and also has high level of safety.

Means for Solving the Problem

The present inventors conducted extensive research to achieve the above object, and found that when enclosing an ionic physiologically active substance as the useful substance in the internal aqueous phase, a high encapsulation ratio in the internal aqueous phase can be achieved by further adding a compound having a molecular weight of 1,000 or less and generating polyvalent counterions in the internal aqueous phase to form a water-soluble complex of the physiologically active substance and the counterions. The inventors also found that the obtained W/O/W emulsion composition has a high encapsulation ratio of useful substances in the internal aqueous phase, excellent safety, and high functionality as an injectable solution. The present invention has been accomplished based on these findings.

More specifically, the present invention provides the W/O/W emulsion compositions described below.

Item 1. A W/O/W multiple emulsion composition composed of an internal aqueous phase, an oil phase, and an external aqueous phase, the internal aqueous phase comprising an ionic physiologically active substance and a physiologically acceptable compound having a molecular weight of 1,000 or less and generating a polyvalent counterion with two or more valencies for the ionic physiologically active substance.

Item 2. The W/O/W multiple emulsion composition according to Item 1, wherein the ionic physiologically active substance is an anionic or cationic physiologically active substance having a molecular weight of 1,000 or less.
Item 3. The W/O/W emulsion composition according to Item 2, wherein
the ionic physiologically active substance is a cationic compound of idarubicin hydrochloride, epirubicin hydrochloride, daunorubicin hydrochloride, doxorubicin hydrochloride, pirarubicin hydrochloride, bupivacaine hydrochloride, lidocaine hydrochloride, tobramycin, arbekacin sulfate, amitriptyline hydrochloride, imipramine hydrochloride, clomipramine hydrochloride, carteolol hydrochloride, epinastine hydrochloride, diltiazem hydrochloride, indenolol hydrochloride, propranolol hydrochloride, metoprolol hydrochloride, mexiletine hydrochloride, dopamine hydrochloride, hydralazine hydrochloride, procaterol hydrochloride, azasetron hydrochloride, granisetron hydrochloride, oxybutynin hydrochloride, propiverine hydrochloride, or ticlopidine hydrochloride; and
the physiologically acceptable compound having a molecular weight of 1,000 or less and generating a polyvalent counterion with two or more valencies is an anionic compound generating a polyvalent anion with two or more valencies.
Item 4. The W/O/W emulsion composition according to Item 2, wherein the anionic compound generating a polyvalent anion with two or more valencies is at least one compound selected from the group consisting of sulfuric acid, phosphoric acid, citric acid, adipic acid, succinic acid, tartaric acid, maleic acid, anhydrous sodium pyrophosphate, sodium tripolyphosphate, sodium tetrapolyphosphate, diethylenetriaminepentaacetic acid, calcium disodium edetate, sodium edetate, glycyrrhizic acid, and boric acid.
Item 5. The W/O/W emulsion composition according to Item 2, wherein the anionic compound generating a polyvalent anion with two or more valencies is at least one compound selected from the group consisting of anhydrous sodium pyrophosphate, sodium tripolyphosphate, sodium tetrapolyphosphate, diethylenetriaminepentaacetic acid, calcium disodium edetate, and sodium edetate.
Item 6. The W/O/W emulsion composition according to Item 2, wherein
the ionic physiologically active substance is an anionic compound of sulbenicillin sodium, cromolyn sodium, bromfenac sodium, diclofenac sodium, captopril, fluvastatin sodium, pemirolast potassium, or ascorbic acid; and
the physiologically acceptable compound having a molecular weight of 1,000 or less and generating a polyvalent counterion with two or more valencies is a cationic compound generating a polyvalent cation with two or more valencies.
Item 7. The W/O/W emulsion composition according to Item 6, wherein the cationic compound generating a polyvalent cation with two or more valencies is a metal compound generating a divalent metal ion.
Item 8. The W/O/W multiple emulsion composition according to Item 1, wherein
the ionic physiologically active substance is an anthracycline antitumor antibiotic; and
the cationic compound generating a polyvalent cation with two or more valencies is anhydrous sodium pyrophosphate.

The W/O/W emulsion composition of the present invention is described in detail below.

Internal Aqueous Phase

For the W/O/W emulsion composition of the present invention, an ionic physiologically active substance is used as a drug to be enclosed in the internal aqueous phase.

The type of physiologically active substances is not limited and can be suitably selected in accordance with the intended use of the W/O/W emulsion composition. The ionicity of physiologically active substances is also not limited; either cationic physiologically active substances or anionic physiologically active substances may be used.

Particularly, the W/O/W emulsion composition of the present invention is advantageous to effectively suppress the leakage, which was supposedly difficult to suppress, of ionic physiologically active substances with a molecular weight of 1,000 or less from the internal aqueous phase.

Specific examples of cationic physiologically active substances effectively usable in the present invention include idarubicin hydrochloride, epirubicin hydrochloride, daunorubicin hydrochloride, doxorubicin hydrochloride, pirarubicin hydrochloride, bupivacaine hydrochloride, lidocaine hydrochloride, tobramycin, arbekacin sulfate, amitriptyline hydrochloride, imipramine hydrochloride, clomipramine hydrochloride, carteolol hydrochloride, epinastine hydrochloride, diltiazem hydrochloride, indenolol hydrochloride, propranolol hydrochloride, metoprolol hydrochloride, mexiletine hydrochloride, dopamine hydrochloride, hydralazine hydrochloride, procaterol hydrochloride, azasetron hydrochloride, granisetron hydrochloride, oxybutynin hydrochloride, propiverine hydrochloride, ticlopidine hydrochloride, and the like. Specific examples of anionic physiologically active substances include sulbenicillin sodium, cromolyn sodium, bromfenac sodium, dichlofenac sodium, captopril, fluvastatin sodium, pemirolast potassium, ascorbic acid, and the like. Among these, anthracycline antitumor antibiotics are particularly preferable since the complex formed with a complex-forming component, described later, is in gel-state at a low temperature, and thus leakage from the internal aqueous phase is

physically suppressed. Specific examples of such anthracycline antitumor antibiotics include idarubicin hydrochloride, epirubicin hydrochloride, daunorubicin hydrochloride, doxorubicin hydrochloride, pirarubicin hydrochloride, and the like.

The amount of an ionic physiologically active substance is not limited; however, in terms of the concentration in the internal aqueous phase, it is preferably about 0.05 to 10 w/v%, and more preferably about 1 to 3 w/v%. Note that the term "w/v%" means the weight (g) of an ionic physiologically active substance in 100 ml of solution.

In the present invention, for the formation of a water-soluble complex of the ionic physiologically active substance, a physiologically acceptable ionic compound having a molecular weight of 1,000 or less and generating polyvalent ions with two or more valencies as counterions for the ionic physiologically active substance (hereinafter also referred to as "complex-forming component") is added to the internal aqueous phase. More specifically, when a cationic physiologically active substance is used, an anionic compound generating polyvalent anions with two or more valencies is added as a complex-forming component; whereas when an anionic physiologically active substance is used, a cationic compound generating polyvalent cations with two or more valencies is added as a complex-forming component.

When such complex-forming components are used, polyvalent ions with two or more valencies are generated, and thus complexes having three or more molecules are formed. Thus, the increased molecular weight suppresses leakage from the internal aqueous phase, raising the encapsulation ratio. Moreover, ionic compounds with a molecular weight of 1,000 or less can be easily transmitted from the internal aqueous phase to the external aqueous phase, thereby decreasing the concentration of complex-forming components unused for the formation of a complex with the ionic physiologically active substance in the internal aqueous phase.

A water-soluble complex can be formed with the ionic physiologically active substance by adding the above-described ionic compound having a molecular weight of 1,000 or less and generating polyvalent ions with two or more valencies, to the internal aqueous phase. The water-soluble complex that is formed is stable in the internal aqueous phase. Additionally, leakage to the external aqueous phase is suppressed, thus ensuring a high encapsulation ratio.

Examples of anionic complex-forming components that satisfy the above-mentioned requirements include sulfuric acid, phosphoric acid, citric acid, adipic acid, succinic acid, tartaric acid, maleic acid, anhydrous sodium pyrophosphate, sodium tripolyphosphate, sodium tetrapolyphosphate, diethylenetriaminepentaacetic acid, calcium disodium edetate, sodium edetate, glycyrrhizic acid, boric acid, etc.

As cationic complex-forming components, compounds forming divalent metal ions such as calcium, magnesium, etc., are preferably used. Examples of such compounds include calcium chloride, magnesium chloride, calcium sulfate, strontium chloride, barium chloride, etc.

These complex-forming components can be used singly or in combination of two or more.

Among these, particularly, anhydrous sodium pyrophosphate, sodium tripolyphosphate, sodium tetrapolyphosphate, diethylenetriaminepentaacetic acid, calcium disodium edetate, sodium edetate, etc. have actually been used as additives in injectable solutions. The W/O/W emulsion composition of the present invention obtained through the use of these complex-forming components is believed to be particularly suitable for injection applications.

Among the foregoing complex-forming components, particularly when using an anthracycline antitumor antibiotic as an ionic physiologically active substance and an anhydrous sodium pyrophosphate as a complex-forming component, a water-soluble complex can be formed; additionally, the formed complex is in a sol state at room temperature and in a gel-state under refrigeration (about 5°C or below). Therefore, emulsion compositions produced at room temperature can be stored under refrigeration to prevent decline in the encapsulation ratio of the anthracycline antibiotic over time.

The amount of such complex-forming components is not limited; however, it is preferably, in terms of the concentration in the internal aqueous phase, about 0.1 to 5 w/v%, and more preferably about 0.5 to 1.5 w/v%. Regarding the proportion of an ionic physiologically active substance to a complex-forming component, it is preferable to use about 0.5 to 2 mol of the complex-forming component with respect to 1 mol of the ionic physiologically active substance.

The pH of the internal aqueous phase of the W/O/W emulsion composition of the present invention is not particularly limited. However, when a cationic physiologically active substance is used, the pH is preferably equal to or below the pKa of the substance; when an anionic physiologically active substance is used, the pH is preferably equal to or above the pKa of the anionic physiologically active substance.

## Oil Phase

For the W/O/W emulsion composition of the present invention, various natural or synthetic oils conventionally used for emulsion production can be used as oily components of the oil phase. For example, mineral oil, vegetable oil, animal oil, essential oil, synthetic oil, and the esters of these oils, etc. can be used. Particularly preferred are vegetable oils, such as soybean oil, cottonseed oil, rapeseed oil, sesame oil, corn oil, arachis oil, safflower oil, and poppy oil. Examples include Coconad (trade name; Kao), ODO (trade name; Nisshin Oil Mills), Migriol (trade name; Mitsuba Trading), Panacete (trade name; Nippon Oil & Fats), and like commercially available medium chain triglycerides. Particularly, commercially available ethyl ester of iodinated poppy-seed oil fatty acid, such as Lipiodol (trade name; Guerbet),

etc. can be suitably used.

Oily components have properties of easily depositing or accumulating on vascular endothelium, tumor tissue, inflammatory tissue, etc. Thus, oily components should be suitably selected for their potential DDS (drug delivery system) effects and carrier effects according to their purpose. Oily components can be used singly or in combination of two or more.

As surfactants added to the oil phase, known lipophilic surfactants can be used. Particularly preferred are lipophilic surfactants having an HLB value of 6 or less, which are effective to stably increase the proportion of the internal aqueous phase. Examples of suitable lipophilic surfactants having an HLB of 6 or less include polyglyceryl-condensed-ricinoleate (PGCR) represented by the following chemical formula:

Formula 1

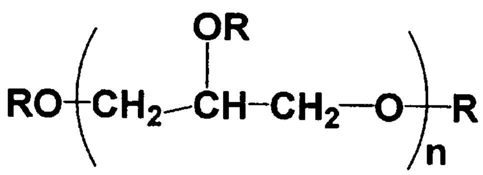

n=(2-8)

wherein R is hydrogen or the following group:

Formula 2

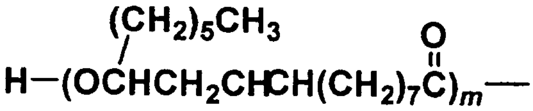

(m=2-30)

Specific examples of such lipophilic surfactants include those commercially available, such as SY Glyster CR-310 (trade name) and SY Glyster CR-500 (trade name; both manufactured by Sakamoto Yakuhin Kogyo), Poem PR-100 (trade name) and Poem PR-300 (trade name; both manufactured by Riken Vitamin), Hexaglyn PR-15 (trade name) and Decaglyn PR-20 (trade name; both manufactured by Nikko Chemicals), Sunsoft 818DG (trade name), Sunsoft 818H (trade name), and Sunsoft 818R (trade name; all manufactured by Taiyo Kagaku), etc.

The amount of a lipophilic surfactant used is not limited as long as a sufficient emulsification effect can be obtained. However it is usually about 0.01 to 0.3 volume, and preferably about 0.05 to 0.15 volume, based on 1 volume of the oily component.

External Aqueous Phase

The external aqueous phase contains a hydrophilic surfactant. Known hydrophilic surfactants can be used; however, those having an HLB value of at least 10 are particularly preferable. Examples of hydrophilic surfactants include Polysorbate 80 (polyoxyethylene sorbitan monooleate) (trade name: Nikkol TO-10M (Nikko Chemicals)), NOFABLE ESO-9920 (trade name; Nippon Oil & Fats), Rheodol TW-0120 (trade name; Kao), Eumulgin SMO20 (trade name; Henkel Japan), Newkalgen D-945 (trade name; Takemoto oil and fat), Adeka Estol T-82 (trade name; Asahi Denka Kogyo), etc.); HCO-60 (polyoxyethylene hydrogenated castor oil) (trade name: Nikkol HCO-60 (Nikko Chemicals)), Uniox HC-60 (trade name; Nippon Oil & Fats), Emanon CH60 (trade name; Kao), Cremophor (trade name; BASF Japan), Eumulgin HRE60 (trade name; Henkel Japan), Emalex HC-60 (trade name; Nihon Emulsion), etc.); and the like.

The amount of a hydrophilic surfactant added is not limited as long as a sufficient emulsification effect can be obtained. Usually, it is preferably, in terms of the concentration in the external aqueous phase, about 0.1 to 5 w/v%, and more preferably about 0.5 to 2 w/v%. Hydrophilic surfactants may be used singly or in combination of two or more.

Other Additives

The W/O/W emulsion composition of the present invention can further contain, if necessary, appropriate amounts of various additives that are known to be containable in this kind of emulsion composition. For example,

antioxidants, antimicrobial agents, pH adjusting agents, isotonizing agents, soothing agents, etc. can be used as additives. Specific examples of antioxidants include sodium metabisulfite (also acts as an antimicrobial agent), sodium sulfite, sodium bisulfite, potassium metabisulfite, potassium sulfite, sodium thiosulfate, and the like. Specific examples of antimicrobial agents include sodium caprylate, methyl benzoate, sodium metabisulfite (also acts as an antioxidant), sodium edetate, and the like. Specific examples of pH adjusting agents include hydrochloric acid, acetic acid, lactic acid, malic acid, citric acid, sodium hydroxide, and the like. Specific examples of isotonizing agents include glycerol; glucose, fructose, maltose, and like saccharides; sorbitol, xylitol, and like sugar alcohols; etc. Among these, oil-soluble materials can be used by previously adding to, for example, oily components that forms the oil phase. Water-soluble materials can be previously added to water (water for injection etc.), used for the preparation of the internal aqueous phase or the external aqueous phase, or added to the obtained emulsion composition and dissolved in its aqueous phase. The amount of these additives added is obvious for a person skilled in the art and does not differ from that conventionally known.

Method of Producing W/O/W Emulsion Composition

The following is one example of a production method of the W/O/W emulsion composition of the present invention.

First, oil phase components including an oily component, a lipophilic surfactant, etc. are put in a container. The container is set in, for example, a high-speed stirring emulsifier, and the mixture is made homogeneous by heating at about 50 to 90°C while stirring. Then, an aqueous phase in which a physiologically active substance, a complex-forming component, and other additives, which are to be added to the internal aqueous phase, are dissolved is gradually added in the container filled with the oil phase components.

Next, while maintaining the temperature in the range of about 50 to 90°C, the mixture is emulsified by stirring to prepare a W/O emulsion. In this W/O emulsion, the ratio of the internal aqueous phase to the oil phase is, in terms of volume ratio of the internal aqueous phase : the oil phase, preferably about 1:0.5-50, and more preferably about 1: 1.5-20. It is desirable to produce the W/O emulsion so as to have an average aqueous-phase particle size of about 0.5 to 10 μm.

Subsequently, the obtained W/O emulsion is dispersed in an external aqueous phase that contains a hydrophilic surfactant. Thus, the W/O/W emulsion composition of the present invention can be obtained.

The W/O emulsion can be dispersed in the external aqueous phase by any standard methods including, for example, a high pressure homogenizer method, a high-speed stirring method, an ultrasonic emulsification method, a membrane emulsification method, etc. In addition, heat can be applied, as required, during the preparation of the W/O/W emulsion composition. The ratio of the W/O emulsion to the external aqueous phase in the W/O/W composite emulsion is, in terms of volume ratio of the W/O emulsion : the external aqueous phase, preferably about 1:0.3-30, and more preferably about 1:0.5-5.

The W/O/W emulsion composition of the present invention obtained in the above-mentioned manner can be processed into solutions, emulsions, creams, and other dosage forms.

Effect of the Invention

The W/O/W emulsion composition of the present invention is obtained by using a physiologically active substance as a drug component, and encapsulating it with a highly safe compound having biocompatibility as a water-soluble complex in the internal aqueous phase. As a highly safe W/O/W emulsion, the composition is a very useful liquid formulation because it has a high encapsulation ratio of drug components in the internal aqueous phase, and can maintain a high encapsulation rate for a long period of time.

Moreover, the release time of the drug components can be controlled by changing the amount of the above compound added to the composition.

BEST MODE FOR CARRYING OUT THE INVENTION

The present invention is described in more detail below with reference to Examples.

Examples 1-13

Preparation of W/O/W Emulsion Composition

Using epirubicin hydrochloride as a drug component, and each compound shown in Table 1, described below, as a complex-forming component, an aqueous solution for internal aqueous phase, which contains 0.1 w/v% epirubicin hydrochloride, 1 w/v% composite-forming component, 4.75 w/v% glucose, and 0.014 M citric acid dissolved in water for

injection, was prepared.

Polyglyceryl-condensed-ricinoleate (PGCR), i.e., a lipophilic surfactant, was dissolved in ethyl ester of iodinated poppy-seed oil fatty acid (trade name: Lipiodol (Guerbet)), i.e., an oily component, to prepare 5.5 mL of oil phase at a PGCR concentration of 10 w/v%. Then, 1 mL of the above aqueous solution for the internal aqueous phase was added thereto, and stirred by a Polytron homogenizer (manufactured by Kinematica) under a nitrogen stream and under heating at 50°C for 10 minutes at 25,000 revolutions per minute. Thus, an emulsion was prepared.

Meanwhile, 6.5 mL of this emulsion was extruded through a hydrophilic porous glass membrane (average pore sizes: 20 μm; manufactured by SPG Technology) into 7.5 mL of aqueous solution for the external aqueous phase, in which 1 w/v% hydrophilic surfactant (polyoxyethylene hydrogenated castor oil; HCO-60) and 5 w/v% glucose were dissolved. Thus, a W/O/W emulsion composition was obtained. As a result of the measurement, the average particle size of the emulsion was 60 μm. The emulsion was stable during refrigerated storage for six months.

Measurement of Aqueous-Phase Leak Ratio

After each emulsion composition obtained in the above-mentioned manner was well shaken, a predetermined amount thereof was collected and centrifugated for 5 minutes at 500 g to separate into an aqueous phase and an oil phase. The total volume and the aqueous phase volume were measured. Thereafter, the drug concentration in the aqueous phase was measured by HPLC, and the percentage of leakage to the aqueous phase was calculated by the following equation:

Percentage of leakage to aqueous phase (%) = aqueous phase concentration x aqueous phase volume / (total volume x concentration in total composition (1%)) x 100

As comparative examples, the percentage of leakage to the aqueous phase without the addition of any complex-forming component was determined. Table 1 shows the ratio (%) of the percentage of leakage to the aqueous phase with the addition of a complex-forming component, to the above-determined percentage of leakage to the aqueous phase without the addition of any complex-forming component as the aqueous-phase leak ratio (%).

Table 1

Drug component: epirubicin hydrochloride

| | Comp. Ex. | Ex. | | | | | |
|---|---|---|---|---|---|---|---|
| | 1 | 1 | 2 | 3 | 4 | 5 | 6 |
| Complex-forming component | Not added | Sulfuric acid | Phosphoric acid | Citric acid | Adipic acid | Succinic acid | Tartaric acid |
| Aqueous-phase leak ratio (%) | 100 | 3.3 | 3.0 | 16.2 | 4.4 | 11.8 | 0.0 |

| | Ex. | | | | | | |
|---|---|---|---|---|---|---|---|
| | 7 | 8 | 9 | 10 | 11 | 12 | 13 |
| Complex-forming component | Maleic acid | Anhydrous sodium pyrophosphate | Sodium tripolyphosphate | Sodium tetrapolyphosphate | Diethylenetriaminepentaacetic acid | Sodium edetate | Glycyrrhizic acid |
| Aqueous-phase leak ratio (%) | 27.7 | 36.8 | 28.8 | 44.8 | 48.1 | 34.3 | 54.4 |

Examples 14-20

Preparation of W/O/W Emulsion Composition

Using doxorubicin hydrochloride as a drug component and each compound shown in the following Table 2 as a complex-forming component, a W/O/W emulsion composition was prepared by the preparation method used in Examples 1-13.

Measurement of Aqueous-Phase Leak Ratio

The aqueous-phase leak ratio (%) was determined by the method used in Examples 1-13.
Table 2

Drug component: doxorubicin hydrochloride

| | Comp. Ex. | Ex. | | | | |
|---|---|---|---|---|---|---|
| | 2 | 14 | 15 | 16 | 17 | 18 |
| Complex-forming component | Not added | Maleic acid | Anhydrous sodium pyrophosphate | Sodium tripolyphosphate | Sodium tetrapolyphosphate | Diethylenetriaminepentaacetic acid |
| Aqueous-phase leak ratio (%) | 100 | 0.0 | 0.0 | 0.0 | 0.0 | 0.0 |

| | Ex. | |
|---|---|---|
| | 19 | 20 |
| Complex-forming component | Sodium edetate | Glycyrrhizic acid |
| Aqueous-phase leak ratio (%) | 0.0 | 0.0 |

Example 21

Preparation of W/O/W Emulsion Composition

Using bupivacaine hydrochloride as a drug component and each compound shown in the following Table 3 as a complex-forming component, a W/O/W emulsion composition was prepared by the preparation method used in Examples 1-13.

Measurement of Aqueous-Phase Leak Ratio

The aqueous-phase leak ratio (%) was determined by the method used in Examples 1-13. The drug concentration was calculated from the absorbance.
Table 3

| Drug component: bupivacaine hydrochloride | | |
|---|---|---|
| | Comp. Ex. | Ex. |
| | 3 | 21 |
| Complex-forming component | Not added | Sodium tripolyphosphate |

(continued)

| Drug component: bupivacaine hydrochloride | | |
|---|---|---|
| | Comp. Ex. | Ex. |
| | 3 | 21 |
| Aqueous-phase leak ratio (%) | 100 | 62.9 |

Example 22

Preparation of W/O/W Emulsion Composition

Using amitriptyline hydrochloride as a drug component and each compound shown in the following Table 4 as a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.
Table 4

| Drug component: amitriptyline hydrochloride | | |
|---|---|---|
| | Comp. Ex. | Ex. |
| | 4 | 22 |
| Complex-forming component | Not added | Glycyrrhizic acid |
| Aqueous-phase leak ratio (%) | 100 | 33.5 |

Examples 23-33

Preparation of W/O/W Emulsion Composition

Using imipramine hydrochloride as a drug component and each compound shown in the following Table 5 as a complex-forming component, a W/O/W emulsion composition was prepared by the preparation method used in Examples 1-13.
The concentration of each complex-forming component added was set at 10%.

Measurement of Aqueous-Phase Leak Ratio

The aqueous-phase leak ratio (%) was determined by the method of Example 21.
Table 5

Drug component: imipramine hydrochloride

| | Comp. Ex. | Ex. | | | | | |
|---|---|---|---|---|---|---|---|
| | 5 | 23 | 24 | 25 | 26 | 27 | 28 |
| Complex-forming component | Not added | Sulfuric acid | Citric acid | Adipic acid | Succinic acid | Anhydrous sodium pyrophosphate | Sodium tripolyphosphate |
| Aqueous-phase leak ratio (%) | 100 | 35.1 | 45.8 | 27.3 | 0.0 | 41.6 | 40.0 |

| | Ex. | | | | |
|---|---|---|---|---|---|
| | 29 | 30 | 31 | 32 | 33 |
| Complex-forming component | Sodium tetrapolyphosphate | Diethylenetriamine pentaacetic acid | Sodium edetate | Glycyrrhizic acid | Boric acid |
| Aqueous-phase leak ratio (%) | 39.8 | 21.0 | 0.0 | 0.0 | 0.0 |

Examples 34-38

Preparation of W/O/W Emulsion Composition

Using clomipramine hydrochloride as a drug component, and each compound shown in the following Table 6 as a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.
Table 6

| Drug component: clomipramine hydrochloride | | | | | | |
|---|---|---|---|---|---|---|
| | Comp. Ex. | Ex. | | | | |
| | 6 | 34 | 35 | 36 | 37 | 38 |
| Complex-forming component | Not added | Sodium tripolyphosphate | Sodium tetrapolyphosphate | Diethylenetriamine pentaacetic acid | Sodium edetate | Glycyrrhizic acid |
| Aqueous-phase leak ratio (%) | 100 | 63.6 | 32.3 | 40.1 | 63.0 | 7.4 |

Examples 39-41

Preparation of W/O/W Emulsion Composition

Using diltiazem hydrochloride as a drug component and each compound shown in the following Table 7 as a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.

Table 7

| Drug component: diltiazem hydrochloride | | | | |
|---|---|---|---|---|
| | Comp. Ex. | Ex. | | |
| | 7 | 39 | 40 | 41 |
| Complex-forming component | Not added | Sodium tetrapolyphosphate | Diethylene triaminepentaacetic acid | Glycyrrhizic acid |
| Aqueous-phase leak ratio (%) | 100 | 55.3 | 43.8 | 26.6 |

Examples 42-52

Preparation of W/O/W Emulsion Composition

Using propranolol hydrochloride as a drug component and each compound shown in the following Table 8 as a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.

Table 8

Drug component: propranolol hydrochloride

| | Comp. Ex. | Ex. | | | | | |
|---|---|---|---|---|---|---|---|
| | 8 | 42 | 43 | 44 | 45 | 46 | 47 |
| Complex-forming component | Not added | Sulfuric acid | Phosphoric acid | Citric acid | Adipic acid | Succinic acid | Maleic acid |
| Aqueous-phase leak ratio (%) | 100 | 23.7 | 19.3 | 19.4 | 23.5 | 16.7 | 55.0 |

| | Ex. | | | | |
|---|---|---|---|---|---|
| | 48 | 49 | 50 | 51 | 52 |
| Complex-forming component | Sodium tripolyphosphate | Sodium tetrapolyphosphate | Diethylenetriaminepentaacetic acid | Sodium edetate | Glycyrrhizic acid |
| Aqueous-phase leak ratio (%) | 63.5 | 18.0 | 12.2 | 21.7 | 7.2 |

Examples 53-58

Preparation of W/O/W Emulsion Composition

Using mexiletine hydrochloride as a drug component and each compound shown in the following Table 9 as

a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.
Table 9

| Drug component: mexiletine hydrochloride | | | | | | | |
|---|---|---|---|---|---|---|---|
| | Comp. Ex. | Ex. | | | | | |
| | 9 | 53 | 54 | 55 | 56 | 57 | 58 |
| Complex-forming component | Not added | Citric acid | Adipic acid | Sodium tetrapolyphosphate | Diethylnetriaminepentaacetic acid | Sodium edetate | Boric acid |
| Aqueous-phase leak ratio (%) | 100 | 19.1 | 30.8 | 21.4 | 32.5 | 18.4 | 19.6 |

Examples 59-68

Preparation of W/O/W Emulsion Composition

Using hydralazine hydrochloride as a drug component and each compound shown in the following Table 10 as a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.
Table 10

Drug component: hydralazine hydrochloride

| | Comp. Ex. | Ex. | | | | |
|---|---|---|---|---|---|---|
| | 10 | 59 | 60 | 61 | 62 | 63 |
| Complex-forming component | Not added | Sulfuric acid | Phosphoric acid | Citric acid | Adipic acid | Succinic acid |
| Aqueous-phase leak ratio (%) | 100 | 17.2 | 23.4 | 35.0 | 42.6 | 48.2 |

Drug component: hydralazine hydrochloride

| | Ex. | | | | |
|---|---|---|---|---|---|
| | 64 | 65 | 66 | 67 | 68 |
| Complex-forming component | Tartaric acid | Sodium tetrapolyphosphate | Diethylenetriaminepentaacetic acid | Sodium edetate | Glycyrrhizic acid |
| Aqueous-phase leak ratio (%) | 39.7 | 47.9 | 18.5 | 52.5 | 22.3 |

Examples 69-70

Preparation of W/O/W Emulsion Composition

Using ticlopidine hydrochloride as a drug component and each compound shown in the following Table 11 as a complex-forming component, a W/O/W emulsion composition was prepared by the preparation method used in Examples 1-13.

Measurement of Aqueous-Phase Leak Ratio

The aqueous-phase leak ratio (%) was determined by the method of Example 21.
Table 11

| Drug component: ticlopidine hydrochloride | | | |
|---|---|---|---|
| | Comp. Ex. | Ex. | |
| | 11 | 69 | 70 |
| Complex-forming component | Not added | Succinic acid | Diethylene triaminepentaacetic acid |

(continued)

| Drug component: ticlopidine hydrochloride | | | |
|---|---|---|---|
| | Comp. Ex. | Ex. | |
| | 11 | 69 | 70 |
| Aqueous-phase leak ratio (%) | 100 | 58.2 | 54.2 |

Examples 71-73

Preparation of W/O/W Emulsion Composition

Using cromolyn sodium as a drug component and each compound shown in the following Table 12 as a complex-forming component, an aqueous solution for the internal aqueous phase, which contains 0.1 w/v% cromolyn sodium, 10 w/v% complex-forming component, and 0.9% sodium chloride dissolved in water for injection, was prepared.

Polyglyceryl-condensed-ricinoleate (PGCR), i.e., a lipophilic surfactant, was dissolved in ethyl ester of iodinated poppy-seed oil fatty acid (trade name: Lipiodol (Guerbet)) i.e., an oily component, to prepare 5.5 mL of oil phase at a PGCR concentration of 10 w/v%. Then, 1 mL of the above aqueous solution for the internal aqueous phase was added thereto, and stirred by a Polytron homogenizer (manufactured by Kinematica) under a nitrogen stream and under heating at 50°C for 10 minutes at 25,000 revolutions per minute. Thus, an emulsion was prepared.

Meanwhile, 6.5 mL of this emulsion was extruded through a hydrophilic porous glass membrane (average pore size: 20 μm; manufactured by SPG Technology) into 7.5 mL of aqueous solution for the external aqueous phase, in which 1 w/v% hydrophilic surfactant (polyoxyethylene hydrogenated castor oil; HCO-60) and 5 w/v% glucose were dissolved. Thus, a W/O/W emulsion composition having an average particle size of 60 μm was obtained.

Measurement of Aqueous-Phase Leak Ratio

The aqueous-phase leak ratio (%) was determined by the method of Example 21.

Table 12

| Drug component: cromolyn sodium | | | | |
|---|---|---|---|---|
| | Comp. Ex. | Ex. | | |
| | 12 | 71 | 72 | 73 |
| Complex-forming component | Not added | Calcium chloride | Magnesium chloride | Barium chloride |
| Aqueous-phase leak ratio (%) | 100 | 22.0 | 19.8 | 55.8 |

Examples 74-75

Preparation of W/O/W Emulsion Composition

Using dichlofenac sodium as a drug component, and each compound shown in the following Table 13 as a complex-forming component, a W/O/W emulsion composition was prepared by the preparation method used in Examples 71-73. The concentration of each complex-forming component added was set at 1%w/v%.

Measurement of Aqueous-Phase Leak Ratio

The aqueous-phase leak ratio (%) was determined by the method of Example 21.

Table 13

| Drug component: dichlofenac sodium | | | |
|---|---|---|---|
| | Comp. Ex. | Ex. | |
| | 13 | 74 | 75 |
| Complex-forming component | Not added | Calcium chloride | Magnesium chloride |
| Aqueous-phase leak ratio (%) | 100 | 61.8 | 46.8 |

Examples 76-78

Preparation of W/O/W Emulsion Composition

Using captopril as a drug component, and each compound shown in the following Table 14 as a complex-forming component, a W/O/W emulsion composition was prepared by the preparation method of Examples 71-73. The concentration of each complex-forming component added was set at 1%w/v%.

Measurement of Aqueous-Phase Leak Ratio

The aqueous-phase leak ratio (%) was determined by the method of Example 21.
Table 14

| Drug component: captopril | | | | |
|---|---|---|---|---|
| | Comp. Ex. | Ex. | | |
| | 14 | 76 | 77 | 78 |
| Complex-forming component | Not added | Calcium chloride | Magnesium chloride | Strontium chloride |
| Aqueous-phase leak ratio (%) | 100 | 62.3 | 56.7 | 64.9 |

Examples 79-82

Preparation of W/O/W Emulsion Composition

Using ascorbic acid as a drug component, and each compound shown in the following Table 15 as a complex-forming component, a W/O/W emulsion composition was prepared by the preparation method used in Examples 71-73.

Measurement of Aqueous-Phase Leak Ratio

The aqueous-phase leak ratio (%) was determined by the method of Example 21.
Table 15

| Drug component: ascorbic acid | | | | | |
|---|---|---|---|---|---|
| | Comp. Ex. | Ex. | | | |
| | 15 | 79 | 80 | 81 | 82 |
| Complex-forming component | Not added | Calcium chloride | Magnesium chloride | Strontium chloride | Barium chloride |
| Aqueous-phase leak ratio (%) | 100 | 5.9 | 4.8 | 0.0 | 39.9 |

As is clear from these results, the W/O/W emulsion composition of the present invention has a high encapsulation ratio of a drug component and excellent stability.

**Claims**

1. A W/O/W multiple emulsion composition composed of an internal aqueous phase, an oil phase, and an external aqueous phase, the internal aqueous phase comprising:

   an ionic physiologically active substance; and
   a physiologically acceptable compound having a molecular weight of 1,000 or less and generating a polyvalent counterion with two or more valencies for said ionic physiologically active substance.

2. The W/O/W multiple emulsion composition according to claim 1, wherein the ionic physiologically active substance is an anionic or cationic physiologically active substance having a molecular weight of 1,000 or less.

3. The W/O/W emulsion composition according to claim 2, wherein
   the ionic physiologically active substance is a cationic compound of idarubicin hydrochloride, epirubicin hydrochloride, daunorubicin hydrochloride, doxorubicin hydrochloride, pirarubicin hydrochloride, bupivacaine hydrochloride, lidocaine hydrochloride, tobramycin, arbekacin sulfate, amitriptyline hydrochloride, imipramine hydrochloride, clomipramine hydrochloride, carteolol hydrochloride, epinastine hydrochloride, diltiazem hydrochloride, indenolol hydrochloride, propranolol hydrochloride, metoprolol hydrochloride, mexiletine hydrochloride, dopamine hydrochloride, hydralazine hydrochloride, procaterol hydrochloride, azasetron hydrochloride, granisetron hydrochloride, oxybutynin hydrochloride, propiverine hydrochloride, or ticlopidine hydrochloride; and
   the physiologically acceptable compound having a molecular weight of 1,000 or less and generating a polyvalent counterion with two or more valencies is an anionic compound generating a polyvalent anion with two or more valencies.

4. The W/O/W emulsion composition according to claim 2, wherein the anionic compound generating a polyvalent anion with two or more valencies is at least one compound selected from the group consisting of sulfuric acid, phosphoric acid, citric acid, adipic acid, succinic acid, tartaric acid, maleic acid, anhydrous sodium pyrophosphate, sodium tripolyphosphate, sodium tetrapolyphosphate, diethylenetriaminepentaacetic acid, calcium disodium edetate, sodium edetate, glycyrrhizic acid, and boric acid.

5. The W/O/W emulsion composition according to claim 2, wherein the anionic compound generating a polyvalent anion with two or more valencies is at least one compound selected from the group consisting of anhydrous sodium pyrophosphate, sodium tripolyphosphate, sodium tetrapolyphosphate, diethylenetriaminepentaacetic acid, calcium disodium edetate, and sodium edetate.

6. The W/O/W emulsion composition according to claim 2, wherein
   the ionic physiologically active substance is an anionic compound of sulbenicillin sodium, cromolyn sodium, bromfenac sodium, diclofenac sodium, captopril, fluvastatin sodium, pemirolast potassium, or ascorbic acid; and
   the physiologically acceptable compound having a molecular weight of 1,000 or less and generating a polyvalent counterion with two or more valencies is a cationic compound generating a polyvalent cation with two or more valencies.

7. The W/O/W emulsion composition according to claim 6, wherein the cationic compound generating a polyvalent cation with two or more valencies is a metal compound generating a divalent metal ion.

8. The W/O/W multiple emulsion composition according to claim 1, wherein
   the ionic physiologically active substance is an anthracycline antitumor antibiotic; and
   the cationic compound generating a polyvalent cation with two or more valencies is anhydrous sodium pyrophosphate.

## INTERNATIONAL SEARCH REPORT

International application No.
PCT/JP2007/070896

A. CLASSIFICATION OF SUBJECT MATTER
*A61K9/113*(2006.01)i, *A61K8/04*(2006.01)i, *A61K8/30*(2006.01)i, *A61K31/135*(2006.01)i, *A61K31/138*(2006.01)i, *A61K31/196*(2006.01)i, *A61K31/352*(2006.01)i, *A61K31/375*(2006.01)i, *A61K31/401*(2006.01)i,

According to International Patent Classification (IPC) or to both national classification and IPC

B. FIELDS SEARCHED

Minimum documentation searched (classification system followed by classification symbols)
A61K9/113, A61K8/04, A61K8/30, A61K31/135, A61K31/138, A61K31/196, A61K31/352, A61K31/375, A61K31/401, A61K31/4365, A61K31/445, A61K31/502, A61K31/55, A61K31/554, A61K31/704, A61K47/02, A61K47/12, A61K47/18, A61K47/26

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 60-100516 A (Takeda Chemical Industries, Ltd.),<br>04 June, 1985 (04.06.85),<br>Claims; pages 2 to 4; page 5, lower left column, line 18 to upper right column, line 10; page 7, upper left column, lines 4 to 9<br>& EP 145240 A & US 4652441 A | 1-5<br>4,5,8 |
| X | JP 2003-175092 A (Canon Inc.),<br>24 June, 2003 (24.06.03),<br>Claims 16 to 19; Par. Nos. [0243], [0335]<br>& EP 1275378 A2 & US 2003/0194443 A1 | 1,2,6,7 |
| Y | JP 2003-104836 A (Kanebo, Ltd.),<br>09 April, 2003 (09.04.03),<br>Par. Nos. [0016], [0018]<br>(Family: none) | 4,5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 November, 2007 (21.11.07) | 04 December, 2007 (04.12.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/070896

C (Continuation). DOCUMENTS CONSIDERED TO BE RELEVANT

| Category* | Citation of document, with indication, where appropriate, of the relevant passages | Relevant to claim No. |
|---|---|---|
| Y | WO 00/076525 A1 (Meiji Milk Products Co., Ltd., Japan Science and Technology Corp.), 21 December, 2000 (21.12.00), Claims; page 5 & AU 200052469 A | 8 |
| Y | JP 2005-97291 A (Taisho Pharmaceutical Co., Ltd.), 14 April, 2005 (14.04.05), Par. Nos. [0009], [0014] (Family: none) | 8 |
| P,Y | JP 2007-99760 A (Ono Pharmaceutical Co., Ltd.), 19 April, 2007 (19.04.07), Par. Nos. [0129], [0139], [0140] (Family: none) | 4,5 |
| A | JP 2003-40765 A (Lion Corp.), 13 February, 2003 (13.02.03), Par. Nos. [0012], [0017] (Family: none) | 1,2,6,7 |

Form PCT/ISA/210 (continuation of second sheet) (April 2007)

**INTERNATIONAL SEARCH REPORT**

International application No.
PCT/JP2007/070896

Continuation of A. CLASSIFICATION OF SUBJECT MATTER
(International Patent Classification (IPC))

*A61K31/4365*(2006.01)i, *A61K31/445*(2006.01)i, *A61K31/502*(2006.01)i, *A61K31/55*(2006.01)i, *A61K31/554*(2006.01)i, *A61K31/704*(2006.01)i, *A61K47/02*(2006.01)i, *A61K47/12*(2006.01)i, *A61K47/18*(2006.01)i, *A61K47/26*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005097292 A